# EUROPEAN PATENT APPLICATION

(11) **EP 1 618 886 A1**
(43) Date of publication of application: **25.01.2006**
(21) Application number: 04017128.2
(22) Date of filing: 20.07.2004
(51) Int. Cl.: A61K 38/02

(54) **Bacteriophage and prophage proteins in cancer gene therapy**

(71) Applicant: Fonds zur Förderung der Forschung auf dem Gebiet der molekularen Virologie und Gentherapie, 1210 Wien (AT)
(72) Inventor: Bläsi, Udo., A 3464 Hausleiten (AT); Hohenadl, Christine., A 1220 Wien (AT)
(74) Representative: Fleuchaus, Andrea

(57) **Abstract**

The present invention relates to the use of a polypeptide having a proliferation inhibitory activity or a cell death inducing activity on animal cells. The invention provides further means and methods to use said polypeptide and the corresponding nucleic acids sequence containing the coding regions of said polypeptide. The invention further relates to vectors expressing said polypeptide, to pharmaceutical compositions and therapeutic methods for treating proliferative disorders or diseases like cancer. Last but not least the invention provides a method for a gene therapeutic approach using said polypeptide having a proliferation inhibitory activity or a cell death inducing activity on animal cells.

## Description

### FIELD OF THE INVENTION

The present invention relates to the use of a polypeptide having a proliferation inhibitory activity or a cell death inducing activity on animal cells. The invention provides further means and methods to use said polypeptide and the corresponding nucleic acids sequence containing the coding regions of said polypeptide. The invention further relates to vectors expressing said polypeptide, to pharmaceutical compositions and therapeutic methods for treating proliferative disorders or diseases like cancer. Last but not least the invention provides a method for a gene therapeutic approach using said polypeptide having a proliferation inhibitory activity or a cell death inducing activity on animal cells.

### BACKGROUND OF THE INVENTION

Development of cancer as a cause of death is a growing problem in populations with good health care and a high percentage of aged people. So far, limited options, like surgery or chemo- and radiotherapy, exist to treat cancer or to cure patients. This is one reason why gene therapy approaches have evolved primarily concentrating on treating cancer.

One gene therapy approach makes use of so-called suicide genes. A suicide gene is a gene whose expression in a cell is lethal for that cell.

Most suicide genes mediate a cell killing effect by coding for viral, bacterial, fungal, plant or human enzymes that convert a chemical substance with low inherent toxicity into a cytotoxic compound [1]. This approach is also referred to as gene-directed enzyme prodrug therapy (GDEPT) or virus-directed enzyme prodrug therapy (VDEPT) if viral vectors are used to deliver the gene.

Most enzymes encoded by such prodrug converting or conditionally toxic suicide genes mediate toxicity through disruption of DNA replication while the expressed product of the suicide gene induces the conversion of a prodrug into its toxic metabolites, which then act on replicating DNA [1, 6]. Some of the best-studied enzymes are the Herpes simplex virus (HSV)-1 thymidine kinase [2], cytosine deaminase [3] from bacteria or yeast, bacterial nitroreductase [56] and the mammalian liver enzyme cytochrome P450 [4, 5]. Once these enzymes are expressed in the target cell, the respective prodrug (ganciclovir, fluorocytosine, CB1954, and cyclophosphamide or ifosfamide, respectively) can be applied systemically and will be activated only in cells, which carry and express the respective suicide gene.

In another approach the plant enzyme gene linamarase (lis) hydrolyses the cyanogenic glucoside substrate, linamarin (lin), into glucose and the cell toxic cyanide [57].

The drawbacks of these approaches are the systemic delivery of the prodrug implicating high doses, which may provoke side effects, especially in the case of cytochrome P450, which is physiologically expressed predominantly in cells of the liver.

Another drawback can be seen in the development of cancer cell resistance, which develops either to the prodrug or to cell killing, which in this kind of therapy is usually due to apoptosis [7, 8] and thus depletion of cells carrying the respective suicide gene.

Another strategy makes use of genes encoding for cytotoxic proteins which have a direct lethal effect on cells such as fusogenic membrane proteins like *Vesicular Stomatitis Virus* (VSV) glycoprotein or *Gibbon Ape Leukemia Virus* (GALV) envelope protein [9-11]. Fusogenic membrane proteins, once expressed in a target cell, cause membrane fusions of neighbouring cells leading to the formation of large multinuclear syncytia, which finally die either by apoptosis or necrosis [12-14]. Other toxins that have been used for direct cell killing are e.g. *Corynebacterium diphtheria* toxin A [15-18], Cholera toxin B [19, 20], Anthrax *(Bacillus anthracis)* toxin [21-24], Pseudomonas *(Pseudomonas aeruginosa)* toxin [58], or Ricin *(Ricinus communis)* [58].

Still another approach makes use of so called apoptosis inducers to activate apoptotic pathways and to induce cell death. The genes used in these studies are e.g. caspase-1/interleukin-1β-converting enzyme (ICE) [59], caspase-3/CPP32β [60], caspase-6 [61], caspase-8 [62], Fas associated protein with death domain (FADD) [63] and Bax [64].

Suicide genes must be introduced into cells in ways that ensure their uptake and expression by as many target cells as possible, while limiting their expression by non-target cells. Suicide gene therapy for cancer ideally requires a vector having the capacity to discriminate between target and non-target cells.

Considering viral vectors (for review see [25]), a tightly controlled production system is required when toxic genes are encoded to protect the respective packaging cells [26] from premature killing.

Although some cytotoxic suicide genes are already known, there is still a need for further and alternative suicide genes having advantageous characteristics, particularly having the capacity to kill or eradicate target cells, especially cancer cells in a patient.

It is thus an object of the present invention to provide a new set of cytotoxic suicide genes and their corresponding cytotoxic proteins or polypeptides having a cell growth inhibitory and/or cell death inducing activity in eukaryotic, particularly animal or mammalian cells including human cells.

### DETAILED DESCRIPTION OF THE INVENTION

Here we show that bacteriophage- and pro-phage-derived alpha-helix-type channel forming proteins, in particular holin proteins, which naturally cause lysis of prokaryotic cells through pore formation in the cytoplasmic membrane, causes a significant cell growth inhibitory or even a cell death inducing activity when introduced and expressed in eukaryotic cells, particularly animal or human cells.

In the context of the invention the term "animal" or "animal cells" is used for organism or cells of organism belonging to the kingdom of Animalia according to the current five kingdom system of classification (Margulis, L. & Schwartz, K.V. 1998. *Five Kingdoms. An Illustrated Guide to the Phyla of Life on Earth.,* W.H. Freeman, New York) which comprises the kingdoms Animalia, Plantae, Protista, Fungi and Monera (comprising the prokaryotic bacteria and cyanobacteria). Accordingly, the term "animal cells" also comprises "human" cells. However, for reasons of intensification in several occasions during the application human cells are additionally mentioned.

Bacteriophage-derived "holins" belong to a functional superfamily consisting of at least sixteen distinct families of proteins that exhibit common structural and functional characteristics. More particular, bacteriophage-encoded holins are members of the α-helix type of channel-forming proteins and are required for the lysis of the infected bacterial cells and as a consequence, for the release of bacteriophage particles. However, the members of that family do not exhibit a statistically significant sequence similarity or homology.

In the context of the present invention the term "bacteriophage" comprises bacteriophages per se as well as pro-phages, which are bacteriophages that are in a dormant state or which are even defective. Its genome is either integrated into that of the host bacterium, or is replicated autonomously.

Holins are encoded by genes of pro-phages of Gram-positive and Gram-negative bacteria as well as bacteriophages associated with these organisms [28]. The primary function of holins appears to be the transport of murein hydrolases across the cytoplasmic membrane to the cell wall where these enzymes hydrolyze bonds in the peptidoglycan cell wall polymer as a prelude to cell lyses. When chromosomally encoded, these enzymes are therefore autolysins. Holins may also facilitate leakage of electrolytes and nutrients from the cell cytoplasm, thereby promoting cell death (Martin Lopez & Garcia, 1998). Murein hydrolases lack N-terminal signal sequences, and therefore are not believed to be transported via the general secretory pathway. Holins undoubtedly form oligomeric complexes that generate pores in the cytoplasmic membrane. These pores are thought to provide a passive transport pathway for their substrate proteins.

It was highly surprising for the inventors to find out, that such holins, when they are expressed in human or animal cells induce cell killing or inhibit cell growth in these cells. These results were particularly surprising, since eukaryotic cell metabolism is completely different to the metabolism of bacterial systems. Additionally also the structure and organization of an eukaryotic cell is fundamentally different from a bacterial cell and moreover eukaryotic cells are not hosts for bacteriophages. Accordingly, it was not predictable, whether holins could be expressed in an animal cell or human cell, what pathways they would use, accordingly where in the cellular compartments such proteins would be expressed, whether their functionality would be preserved or modified, whether the cellular metabolism would be affected or activated, and last but not least what effects the introduction and expression of such holins in an eukaryotic cell would have.

While answering these and further basic research questions the inventors were able to provide means and methods to use the bacteriophage-derived holins for treating proliferating diseases and cancer. In particular they provide means and methods for a gene therapeutical approach of treating cancer.

In a preferred embodiment of the invention the bacteriophage-derived proteins are alpha-helix-type channel forming proteins. These proteins are selected, but not limited to double stranded DNA bacteriophages, single stranded DNA bacteriophages or single stranded RNA bacteriophages, more preferably they are selected from the group consisting of the Lambda S105 and Lambda S107 protein (Swiss-Prot Accession Number P03705), the Phi29 GP 14 protein (Swiss-Prot Accession Number P11188), the phage T4 Immunity protein (Swiss-Prot Accession Number P08986), the phage P22 protein 13 (Swiss-Prot Accession Number P09962), the phage P2 TM protein Y (Swiss-Prot Accession Number P51773), the M protein of phage PRD1 (Swiss-Prot Accession Number P27389), the holin protein of phage A118 (Swiss-Prot Accession Number Q37975), the phage A500 holin protein (Swiss-Prot Accession Number Q37977), the DPH holin protein of phage Dp-1 (Swiss-Prot Accession Number 003978), the holin protein of phage HP1 (Swiss-Prot Accession Number P51727), the holin protein of phage rlt (Swiss-Prot Accession Number Q38134), the lysis protein 17.5 of phages T7 (Swiss-Prot Accession Number P03802) and T3 (Swiss-Prot Accession Number P10307), the NucE holin protein of phage P2 Ogr (Swiss-Prot Accession Number Q54418), the holin protein isolated from *Haemophilus somnus* (Swiss-Prot Accession Number Q48281), the P10 protein of phage Phi-6 (Swiss-Prot Accession Number P11127), the protein rV of phage T4 (Swiss-Prot Accession Number P06808), the *Staphylococcus* phage Phi-11 holin (Swiss-Prot Accession Number Q38020), and the *Lactococcus* phage Tuc2009 holin (Swiss-Prot Accession Number Q38613).

Further preferably they are selected from the group having the SEQ ID No. 7, SEQ ID No. 8 and SEQ ID No. 9 (Table No. 2).

Included are also "analoga" of the holin proteins. Analoga that substantially correspond to holins are those polypeptides with conservative changes or in which one or more amino acids of the amino acid sequence of the holin has been replaced with another amino acid, deleted and/or inserted, provided that the resulting protein exhibits substantially the same or even higher biological activity as the holin to which it corresponds.

"Conservative" changes are those changes which would not be expected to change the activity, charge or configuration of the protein and thus would not be expected to change the biological properties thereof. Conservative substitutions include an "analog" wherein at least one amino acid residue in the polypeptide has been conservatively replaced by a different amino acid. Suitable substitutions may be determined by routine experimentation to provide modified structural and functional properties of a synthesized polypeptide while maintaining the biological activity of the holin protein. One or more substitutions could preferably be created in accordance with the following list:
Ala (Gly, Ser); Arg (Lys); Asn (Gln, His); Asp (Glu); Cys (Ser); Gln (Asn); Glu (Asp); Gly (Ala, Pro); His (Asn, Gln); Ile (Leu, Val); Leu (Ile, Val); Lys (Arg, Gln, Glu); Met (Leu, Tyr, Ile); Phe (Met, Leu, Tyr), Ser (Thr); Thr (Ser); Trp (Thr); Tyr (Trp, Phe); and Val (Ile, Leu)
At the genetic level these analoga are generally prepared by site-directed mutagenesis of nucleotides in the DNA encoding the holin protein as described in e.g. Sambrook *et al.,* Molecular Cloning: A Laboratory Manuel, CSH Laboratory, Cold Spring Harbor, N.Y., 1989.

Double stranded DNA (dsDNA) bacteriophages, such as phage Lambda, use a dual system for host cell lysis consisting of (i) a muralytic enzyme or endolysin with enzymatic activities against the glycosidic, amide, or peptide bonds of the peptidoglycan and (ii) a pore-forming holin that allows the endolysin that lacks any secretory signal sequences and accumulates in the cytoplasm to gain access to the peptidoglycan in a timed manner, eventually resulting in of the cell [28, 29]. The holin function is non-specific for the endolysin since artificial lysis systems consisting of a holin and an endolysin originating from distinct bacteriophages have been shown to be lytically competent [29]. This also demonstrates the functional homology of the different holin proteins having distinct amino acid sequences and thus showing no homology on the DNA or amino acid level. Although holins encoded by dsDNA bacteriophages are divergent, they generally exhibit common features: (i) small sizes (60-145 amino acids), (ii) two, three, or four transmembrane-spanning domains, (iii) a hydrophilic N-terminus, and (iv) a highly polar, charge-rich C-terminal domain. The N- and C-terminal hydrophilic domains are known to function in the timing of the holin-mediated channel formation [30, 31]. Despite these common features, holins of dsDNA bacteriophages can be grouped into two general classes. Class I holins, of which S of bacteriophage λ (Lambda) (S^{λ}) is the prototype, are in general of a size of 95 amino acid residues or longer and harbor three potential membrane-spanning domains. Class II holins, such as S²¹ of phage 21 are usually smaller, ranging in size between 65 and 95 amino acid residues, and contain only two transmembrane-spanning domains.

Holins of dsDNA bacteriophages form oligomeric complexes [32, 33]. Only few holin molecules are necessary to lyse a bacterial cell. For S^{λ} it has been shown that holin is lethal for an *E. coli* cell at concentrations of 1-3 x 10³ molecules per cell [34]. Usually, a lysogenic bacterial culture undergoes lysis within minutes after onset of lysis. This precise temporal regulation likely involves the energy state of the membrane since adding an energy poison such as cyanide or dinitrophenol sufficiently late in the infective cycle can instantly trigger premature lysis of cells infected with bacteriophage λ or other bacteriophages [29]. For bacteriophage λ, the exact timing of host cell lysis is controlled by the molar ratio between the actual holin and a holin inhibitor (usually about 2:1) [35]. Both proteins are encoded by the same open reading frame (ORF). The actual holin (S105) originates from translational initiation at codon number 3. The holin inhibitor (S107) is only two amino acid residues longer and is a product of translational initiation at codon number 1. This dual-start motif (i.e. two in-frame ATG start codons separated by one or two codons of which at least one is positively charged [35] is not only found in the S^{λ} ORF but also in a number of other holin genes. The presence of a holin/holin-inhibitor pair has not only experimentally been demonstrated for S^{λ} but also for other phage-encoded lysis systems [30, 36, 37], which are incorporated herein by reference.

In terms of bacterial cell lysis, in the most attractive model [38], holins exist in two conformational states in the cytoplasmic membrane: a "pre-hole" state in which the holin accumulates in the membrane, and the "hole" state in which the actual hole is formed. As the number of holin proteins in the membrane increases, holin dimers accumulate which are the building materials for higher-order oligomers, which eventually fuse to small holin islands. These holin arrays represent instable regions in the membrane and it is thought, that at some point, thermal fluctuation opens a hole within the holin arrays leading to a local depolarization of the membrane. This local depolarization then triggers the conformational change of the surrounding holin proteins from the pre-hole state to the hole state resulting in larger holes and a complete collapse of the membrane potential. In a chain reaction, the breakdown of the membrane potential would trigger the conformational change of more and more holin proteins into the active conformation, eventually leading to the creation of large holes in the cytoplasmic membrane. The hole itself has been shown to be of irregular size but must at least be large enough to allow the endolysin to pass through. Wang *et al.* have shown that the S^{λ} hole in the cytoplasmic membrane of *E. coli* is even large enough to allow the release of a 480 kDa endolysin/LacZ fusion protein [39].

In contrast to dsDNA bacteriophages, simple lytic bacteriophages such as the ssDNA bacteriophages (represented by φX174, α3, G4, S13) and the ssRNA bacteriophages (represented by MS2, GA, Qβ, SP) harbor only a single lysis gene coding for a small integral membrane protein. The lysates of these bacteriophages have been shown to be free of any muralytic enzyme activity and the amino acid sequences of the known holins from these bacteriophages do not indicate such a function either. Expression of plasmid-encoded φX174 lysis gene E in E. *coli* alone is sufficient for efficient cell lysis [40-42]. However, in this case, cell lysis is dependent on actively growing cells [43, 44]. Furthermore, it has been shown, that the bacterial host gene *sly*D coding for a peptidyl-prolyl cis-trans isomerase (PPlase) is absolutely required for cell lysis by wild type protein E [45]. SlyD is probably involved in the correct folding and stabilization of protein E or its delivery to the cytoplasmic membrane [46].

The present invention describes the toxic effect of the bacteriophage holin proteins, exemplarily using the phage λ-encoded S105 protein, when expressed in animal cells, in particular in human cells, e.g. HeLa ,cells, MCF7 cells and 293 cells. Thus, the present invention shows the cytotoxic effect of S105 by describing the morphological changes of the cells after induction of *S105* expression, the cytotoxic effect on membrane integrity (i.e. by trypan blue staining, annexinV-PE/7AAD staining), and the cytotoxic effect on cellular metabolism (i.e. measuring NADPH or NADH levels generated by dehydrogenases, ATP levels or the membrane potential of mitochondria). It was shown that the expression of bacteriophage holin proteins induces a cell growth inhibitory activity and cell killing effect in animal, especially human cells. This cell growth inhibitory activity and cell killing effect is particularly useful for limitating the growth activity of cancer cells or for eradicating cancer cells in a patient and thus for the treatment of cancer.

For the use according to the present invention the Bacteriophage-derived proteins, e.g. holins, need to be expressed in an eukaryotic, especially an animal or a human cell. For this purpose the nucleic acid sequence, e.g. DNA, cDNA, RNA of the phage-derived proteins or functional fragments thereof, or the expression product thereof, needs to be transferred into the cell. Alternatively, the nucleic acid sequence, e.g. RNA or mRNA encoding the holins, needs to be directly synthesised within the cell.

A "nucleic acid sequence" according to the present invention comprises any nucleic acid sequence - e.g. DNA, cDNA, RNA, mRNA -, which encodes for bacteriophage-derived proteins or polypeptides having a holin-like function, e.g. holins, or biological active portions or analoga thereof. This definition embraces also nucleic acid sequences encoding the same polypeptide or protein but differing from the natural sequence due to changes permitted by the known degeneracy of the genetic code. And this definition also includes those nucleic acids capable of hybridizing, under stringent hybridization conditions, to the claimed nucleic acid sequences, including mRNA, cDNA, genomic DNA and functional fragments thereof. Especially a nucleic acid sequence encoding for a fusion protein and a shuttle protein (e.g. HIV-1-Tat, HBV-PreS2-TLM, Sox10, P22) are also included into the definition.

As used herein, the term "stringent hybridization conditions" refers to conditions for hybridization and washing under which nucleotide sequences typically remain hybridized to each other. Such stringent conditions are known to those skilled in the art and can e.g. be found in Current Protocols in Molecular Biology, John Wiley & Sons, N.Y. (1989), 6.3.1.ff. A variety of modifications can be made to DNA and RNA and thus, the term "nucleic acid" also embraces chemically, enzymatically, or metabolically modified forms. It is self explanatory that a "nucleic acid" which hybridizes only to polyA+ sequences like any 3' terminal polyA+ tract of a cDNA, or to a complementary stretch of T (or U) residues, would not be included in the definition of "nucleic acid," since such a nucleic acid would hybridize to any nucleic acid molecule containing a poly (A) stretch or the complement thereof.

For the transfer of nucleic acid sequences into a cell several known vector systems, viral vectors or also naked DNA could be used. In general vectors according to the present invention are DNA vehicles of linearised or circular structure, such as plasmids, cosmids or artificial chromosomes, which in addition to the desired nucleic acid sequence contain regulatory sequences, optionally selective marker genes and optionally replicons enabling the autonomous replication of the vector.

Several methods are known and can be applied to transfer nucleic acid sequences into target cells for the generation of transiently or stably transfected cells. These methods include physical and chemical methods such as electroporation, microinjection, microprojectile bombardment, antibody- or ligand-, dextran-, liposomal-, virosomal-, calcium phosphate-, charged polymer- or charged lipid-mediated DNA transfer. Chemical modifications e.g. derivatisation of peptides, proteins and oligonucleotides with lipophilic structures, may also be used to enhance the uptake into target cells. For instance, the sequence of a known membranotropic peptide may be fused to the nucleic acid sequence as mentioned above, or chemically modifications known to a person skilled in the art could be made to the holins, such as adding lauroyl derivatives, or oxidation of methionine residues to create sulfoxide groups, or replacing the peptide bond by its ketomethylene isoesters to enhance the membrane permeability. Alternatively, molecules that are known to bind to cell surface receptors could be conjugated to such polypeptides, proteins or nucleic acid sequences. Useful examples are sugars, vitamins, hormones, cytokines, transferrin, asiaglycoprotein as described in US 5,108,921, incorporated herein by reference.

According to a preferred embodiment the present invention uses and provides viral vector systems for the transduction of the coding information of the phage-derived holins or biologically active fragments thereof into the eukaryotic cell. In general every viral vector system, which allow the transfer and the subsequent expression of a nucleic acid sequence can be used according to the present invention. Particularly useful are poxviral expression vectors, adenoviral expression vectors, herpes virus expression systems, adeno-associated virus vectors, alphavirus vectors, SV40 vectors, vesicular stomatitis virus vectors, measles virus vectors, sindbis virus vectors, rabies virus vectors, as well as lentiviral and retroviral vector systems.

In a further preferred embodiment retroviral vector systems are used. Such retroviral vector systems are based either on replication competent (e.g. as described in WO018240 and incorporated herein by reference) or on replication deficient retroviral vector systems. While a replication competent retroviral vector system has the advantage of a high transduction rate, it also bears the risk of unspecific transduction. Specificity of gene expression needs therefore to be controlled by specific regulatory elements, such as cell-specific promoters.

In comparison to this, replication deficient retroviral vector systems, like the promoter conversion vector (ProCon) as described in EP0779929B1 or a reconstituting viral (ReCon) vector as described in WO 99/35280 (both incorporated herein by reference), show high cell specificity but are less effectively transducing cells. Since various retroviral vector systems are well described in the art, it is easy for a person skilled in the art to choose a suitable vector system.

In general retroviral vector systems consist of two components, whereby the first component, the retroviral vector itself is a modified retrovirus (vector plasmid) in which the genes encoding for the viral proteins have been replaced by the nucleic acid sequence of interest optionally including marker genes to be transferred to the eukaryotic cell. Since the replacement of the genes coding for the viral proteins effectively cripples the virus, it must be rescued by the second component of the system, which encodes the missing viral proteins. The second component is a cell line that produces large quantities of the viral structural and enzymatic proteins, however lacks the ability to produce replication competent virus. This cell line is known as the packaging cell line and consists of a cell line transiently or stably transfected with one or more plasmids carrying the genes enabling the modified retroviral vector to be packaged. These plasmids direct the synthesis of the necessary viral proteins required for virion production. To generate viral particles or the packaged vectors, the first component or the vector plasmid is transferred into the packaging cell line. Under these conditions the modified retroviral genome including the inserted therapeutic and optional marker genes is transcribed from the vector plasmid and packaged into the modified retroviral particles (recombinant viral particles). A cell infected with such a recombinant viral particle cannot produce new vector virus since no viral proteins are present in these cells. However, the vector carrying the therapeutic and marker genes is present and these can now be expressed in the infected cell.

In another preferred embodiment, a reconstituting viral vector, as described in WO 99/35280, which is incorporated herein by reference, could be used. In this vector system genetic reshuffling during the viral life cycle is exploited, allowing reconstitution of functional expression cassettes from separated elements exclusively in transduced target cells [52]. For this purpose, a heterologous regulatory element or promoter is inserted in the inverse orientation into the unique 5' region (U5) of the long terminal repeat (5' LTR) of the retroviral vector. The therapeutic toxic gene is inserted in the reverse orientation into the unique 3' region (U3) of the 3' long terminal repeat (3'LTR). After reverse transcription, both unique regions are duplicated, reconstituting two functional and active expression cassettes within each of the retroviral LTRs. The use of tissue-specific promoters may ensure expression of the transduced gene only or preferentially in the respective target cells or cells of specific tissues. All promoters can additionally be either constitutive or conditionally active and of viral or non-viral origin.

As used herein, the term "regulatory element" means a nucleic acid sequence that serves as a promoter, i.e., regulates expression of a nucleic acid sequence operably linked to the promoter. Such "regulatory elements" or "promoters" can control the expression of linked nucleic acid sequences either constitutively or inducible. Examples for inducible promoters are tetracycline-dependent regulatory systems (for review see [66]), heat shock, hormone (e.g. ecdysone, doxycyline, rifampicin), or metal ion regulatory systems. Many systems exhibit a significant level of basal expression. This "leakiness" makes it difficult to use them for the expression of cytotoxic genes. "Leaky promoters" according to the present invention are inducible regulatory elements, which exhibit a basal expression of the linked nucleic acid, without induction. The term "tissue-specific promoter" (TSP) means a regulatory element, which is only or primarily active in specific cells or specific cells of a tissue (e.g probasin promoter, human telomerase reverse transcriptase promoter, human surfactant protein A1 promoter, Ksp-cadherin (Cadherin 16) promoter, mouse mammary tumour virus promoter (MMTV-LTR), whey acidic protein promoter (WAP)).

In another preferred embodiment the promoter conversion vector (ProCon) as described in EP0779929B1 and incorporated herein by reference could be used. According to the ProCon principle, a retroviral vector is constructed in which the 3' U3 region is altered, but the normal 5' U3 structure is maintained; the vector can be normally transcribed into RNA utilizing the normal retroviral promoter located within the 5' U3 region. However the generated RNA will only contain the altered 3' U3 structure. In the infected target cell, after reverse transcription, this altered U3 structure will be placed at both ends of the retroviral structure.

If the altered region carries a polylinker instead of the U3 region then any promoter, including those directing tissue-specific expression can be easily inserted. This promoter will then be utilized exclusively in the target cell for expression of linked genes carried by the retroviral vector and can if desired be the only promoter in the vector. Alternative as well as additional DNA segments homologous to one or more celluar sequences can be inserted into the polylinker for the purposes of gene targeting.

In the packaging cell line the expression of the retroviral vector is regulated by the normal unselectively active retroviral promoter. However as soon as the vector enters the target cell, promoter conversion occurs and the therapeutic genes are expressed from a tissue-specific promoter of choice introduced into the polylinker. Not only can virtually any tissue-specific promoter be included in the system, providing for the selective targeting of a wide variety of different cell types, but additionally, following the conversion event, the structure and properties of the retroviral vector no longer resembles that of a virus. This, of course, has extremely important consequences from a safety point of view, since ordinary or state of the art retroviral vectors readily undergo genetic recombination with the packaging vector to produce potentially pathogenic viruses. Promoter conversion (ProCon) vectors do not resemble retroviruses because they no longer carry U3 retroviral promoters after conversion thus reducing the possibility of genetic recombination. Also ProCon vectors, in constrast to ordinary state of the art vectors, may be less likely to activate cellular genes in the vicinity of the integration sites.

In the context of the present invention and to allow the evaluation of a potential toxic effect of the expressed gene a tightly controlled gene expression system is used. A tight control of expression of the toxic gene is particularly important when using viral vector systems, wherein the viral vector needs to be packaged by a packaging cell and, of course, the expression of the toxic gene in the packaging cell would kill said cell. Only after the viral vector has been packaged into a viral particle, and only after the viral particle has infected its host, preferably a eukaryotic cell, more preferably an animal cell, particularly a mammalian or human cell, the toxic gene should be expressed in the host cell.

This could be achieved by e.g. using tight controlled inducible promoter operably linked to the cytotoxic gene. In a tightly controlled system the gene expression can be induced by adding the inducer. Without the inducer the system will be silent and no gene expression occurs. Accordingly, by adding the inducer only after the viral particle had infected the host cell it can be secured that expression of the toxic gene only occurs in the host cell. Examples for inducible promoters are the lac repressor/operator, FK506/rapamycin, ecdysone-inducible, RU486/mifepristone, and tetracycline (Tc)-inducible systems or the mouse mammary tumor virus promoter (MMTV-LTR).

The present invention, in a further embodiment, identifies the value of λ S105, λ. S107, and Φ29 GP14 as toxic proteins for cancer gene therapy using (i) a hormone- inducible gene expression system based on retroviral vectors using the mouse mammary tumor virus (MMTV) promoter that can be activated by glucocorticoids [65] and (ii) a tetracycline/doxycycline inducible gene expression system based on a plasmid vector [67]. Using these inducible gene expression systems for induction a significant effect of the S105 expression could be exemplarily demonstrated on the morphology, on membrane integrity (i.e. by trypan blue staining, annexinV-PE/7AAD staining), and on cellular metabolism (*i*.*e*. measuring NADPH or NADH levels generated by dehydrogenases or measuring ATP levels or measuring membrane potentials) of HeLa cells, MCF-7 cells or 293 cells (see Examples). Thus, it was unexpectedly shown that the expression of bacteriophage-derived holins in human cells leads to a reduction in cell growth and also specifically kills eukaryotic cells, especially animal cells or more specifically mammalian cells including human cells.

According to another embodiment also conventional regulatory elements could be used. Basal transcription from leaky regulatory elements could be silenced by expressing within the packaging system a nucleic acid sequence at least partially complementary to the transcript of the cytotoxic gene (antisense RNA) but still capable of efficiently binding to the transcript of the cytotoxic gene and therefore inhibiting translation of the cytotoxic gene in a way that at least the vitality of the packaging system is prolonged and sufficient viral vectors particles are produced.

The invention, according to still a further embodiment, provides a method to introduce and express the bacteriophage-derived protein, which induces a cell growth inhibitory activity or cell killing effect, or at least the functional part thereof in a eukaryotic cell. For this the nucleic acid sequence encoding the bacteriophage-derived protein or at least the functional part or analoga thereof and an operably linked regulatory element is introduced into the eukaryotic cell, where it subsequently will be expressed under the control of a suitably linked regulatory element. For uptake said nucleic acid sequence can be presented as naked DNA to the eukaryotic cell, which will to a certain extent take up the nucleic acid sequence and express the encoded protein. To increase the efficacy plasmids or other DNA vectors could be transfected into the cells using well-known transfection systems, such as e.g. calcium phosphate. Viral vectors, which could be either recombinant viruses or viral particles, would be used to infect the eukaryotic cell and would thereby transfer the nucleic acid sequence into said cells.

The pharmaceutical composition according to the invention may include beside a therapeutically effective amount of the expression vector, the nucleic acid sequence, the viral particle, the eukaryotic packaging cell and/or the bacteriophage-derived protein in general include one or more pharmaceutical acceptable and/or approved carriers, additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycolic acids, polymeric amino acids, amino acid copolymers, lipid aggregates, or the like.

In another embodiment a therapeutically effective amount of expression vector, the nucleic acid sequence, the viral particle, the eukaryotic producer or packaging cell and/or the bacteriophage-derived protein could be encapsulated into semipermeable membranes (capsule), whose permeability allow passage of smaller molecules such as oxygen, nutrients and waste products as well as the desired therapeutic product, but do not allow the transport of molecules larger than a desired critical size into or out of the surrounding environment. Preferably, such a semipermeable membrane is made from electrolyte complexes (e.g. alginate and poly-L-lysine or dextrose sulfate, or more preferably, cellulose sulfate and poly-dimethyldiallylammonium chloride) or other porous structures such as e.g. polyamides, polyethersulfones (PES), chitosan sulfate, hydroxyethyl-methacrylate-methyl-methacrylate, or PAN/PVC.

The capsules preferably used have a variable diameter between 0.01 and 5 mm and are referred as to be microcapsules. But also other cladding bodies such as macrocapsules, hollow fibers, or nanocapsules can be used. As an example microcapsules as described in EP0835137 and incorporated herein by reference could be used.

Within other aspects of the present invention, methods are provided for preparing a composition or preserving an infectious viral particle or recombinant virus. Briefly, infectious viral particles or recombinant viruses which have been purified or concentrated may be preserved by first adding a sufficient amount of a formulation buffer to the media containing the recombinant virus, in order to form an aqueous suspension. The formulation buffer is an aqueous solution that contains a polysaccharide, a high molecular weight structural additive, and a buffering component in water. As utilized within the context of the present invention, a "buffering compound" or "buffering component" should be understood to refer to a substance that functions to maintain the aqueous suspension at a desired pH. The aqueous solution may also contain one or more amino acids and/or optionally a cellular growth factor.

The infectious viral particles or recombinant viruses can also be preserved in a purified form. More specifically, prior to the addition of the formulation buffer, the crude stock may be clarified by passing it through a filter, and then concentrated, such as by a cross flow concentrating system (Filtron Technology Corp., Nortborough, MA). Within one embodiment, DNase is added to the concentrate to digest exogenous DNA. The digest is then diafiltrated to remove excess media components and establish the infectious viral particles or recombinant virus in a more desirable buffered solution. The diafiltrate is then passed over a Sephadex S-500 gel column and a purified particles or virus is eluted. A sufficient amount of formulation buffer is added to this eluate to reach a desired final concentration of the constituents and to minimally dilute the recombinant virus, and the aqueous suspension is then stored, preferably at -70°C or immediately dried. As noted above, the formulation buffer is an aqueous solution that contains a saccharide, a high molecular weight structural additive, and a buffering component in water. The aqueous solution may also contain one or more amino acids and/or optionally a cellular growth factor.

The crude stock of infectious viral particle or recombinant virus can also be purified by an ultracentrifugation step through a sucrose density gradient. This method is known as a standard procedure of virus purification. In general, the crude stock can be purified by making use of the fact that molecules of a particular density centrifuged in a solution of varying density (e.g. 20% - 60% w/v sucrose in 0.1 M Tris-EDTA pH 9.5) will tend to collect in a band at that zone where the density of the molecule and the density of the solution are exactly equal. After centrifugation, different fractions are collected through a hole in the bottom of the centrifugation tube. Superfluous sucrose is washed out by centrifugation in a buffered solution and the infectious viral particles or recombinant viruses are resuspended in a desired buffered solution. A sufficient amount of formulation buffer is then added, as discussed above, to the purified recombinant virus and the aqueous suspension is either dried immediately or stored, preferably at -70°C.

The aqueous suspension in crude or purified form can be dried by lyophilization or evaporation at ambient temperature. Specifically, lyophilization involves the steps of cooling the aqueous suspension below the glass transition temperature or below the eutectic point temperature of the aqueous suspension, and removing water from the cooled suspension by sublimation to form a lyophilized virus. Briefly, aliquots of the formulated recombinant virus are placed into an Edwards Refrigerated Chamber (3 shelf RC3S unit) attached to a freeze dryer (Supermodulyo 12K). A multistep freeze drying procedure as described by Phillips et al. (Cryobiology 18:414, 1981) is used to lyophilize the formulated recombinant virus, preferably from a temperature of -40°C to - 45°C. The resulting composition contains less than 10% water by weight of the lyophilized virus. Once lyophilized, the recombinant virus is stable and may be stored at -20°C to 25°C, as discussed in more detail below.

The aqueous solutions used for formulation, as previously described, are composed of a saccharide, high molecular weight structural additive, a buffering component, and water. The solution may also include one or more amino acids. The combination of these components act to preserve the activity of the infectious viral particle or recombinant virus upon freezing and lyophilization, or drying through evaporation. Although a preferred saccharide is lactose, other saccharides may be used, such as sucrose, mannitol, glucose, trehalose, inositol, fructose, maltose or galactose. In addition, combinations of saccharides can be used, for example, lactose and mannitol, or sucrose and mannitol. A particularly preferred concentration of lactose is 3% - 4% by weight. Preferably, the concentration of the saccharide ranges from 1% to 12% by weight.

The amino acids, if present, function to further preserve viral infectivity upon cooling and thawing of the aqueous suspension. In addition, amino acids function to further preserve viral infectivity during sublimation of the cooled aqueous suspension and while in the lyophilized state. A preferred amino acid is arginine, but other amino acids such as lysine, ornithine, serine, glycine, glutamine, asparagine, glutamic acid or aspartic acid can also be used. A particularly preferred arginine concentration is 0.1% by weight. Preferably, the amino acid concentration ranges from 0.1% to 10% by weight.

In addition, it may be advantageous to use aqueous solutions containing components known to enhance the activity of the cells to be infected. Growth factors or a combination of growth factors, if present in the pharmaceutical composition, are capable of regulating physiological processes of cells, and hence, in the context of the present invention facilitate the uptake and further integration of the recombinant retroviral vector. Representative examples include epidermal growth factor (EGF), melanocyte stimulating hormone (MSH), insulin like growth factor (IGF) and various cytokines, like Interleukin-2, -4 or -10, Interferon alpha or gamma.

The buffering component acts to buffer the solution by maintaining a relatively constant pH. A variety of buffers may be used, depending on the pH range desired, preferably between 7.0 and 7.8. Suitable buffers include phosphate buffer and citrate buffer. A particularly preferred pH of the recombinant virus formulation is 7.4, and a preferred buffer is tromethamine.

In addition, it is preferable that the aqueous solution contains a neutral salt which is used to adjust the final formulated virus to an appropriate iso-osmotic salt concentration. Suitable neutral salts include sodium chloride, potassium chloride or magnesium chloride. A preferred salt is sodium chloride.

Aqueous solutions containing the desired concentration of the components described above may be prepared as concentrated stock solutions.

A particularly preferred method of preserving recombinant viruses in a lyophilized state for subsequent reconstitution comprises the steps of (a) combining an infectious recombinant virus with an aqueous solution to form an aqueous suspension, the aqueous suspension including 4% by weight of lactose, 0.1% by weight of human serum albumin, 0.03% or less by weight of NaCl, 0.1% by weight of arginine, and an amount of tromethamine buffer effective to provide a pH of the aqueous suspension of approximately 7.4, thereby stabilizing the infectious recombinant virus; (b) cooling the suspension to a temperature of from -40°C to -45°C to form a frozen suspension; and (c) removing water from the frozen suspension by sublimation to form a lyophilized composition having less than 2% water by weight of the lyophilized composition, the composition being capable of infecting mammalian cells upon reconstitution. It is preferred that the recombinant virus is replication competent and most preferred that the recombinant virus is replication defective and suitable for administration into humans upon reconstitution.

It will be evident to those skilled in the art given the disclosure provided herein that it may be preferable to utilize certain saccharides within the aqueous solution when the lyophilized virus is intended for storage at room temperature. More specifically, it is preferable to utilize disaccharides, such as lactose or trehalose, particularly for storage at room temperature.

The lyophilized or dehydrated viruses of the subject invention may be reconstituted using a variety of substances, but are preferably reconstituted using water. In certain instances, dilute salt solutions, which bring the final formulation to isotonicity may also be used. In addition, it may be advantageous to use aqueous solutions containing components known to enhance the activity of the reconstituted virus. Such components include cytokines, such as IL-2, polycations, such as protamine sulfate, or other components, which enhance the transduction efficiency of the reconstituted virus. Lyophilized or dehydrated recombinant virus may be reconstituted with any convenient volume of water or the reconstituting agents noted above that allow substantial, and preferably total solubilization of the lyophilized or dehydrated sample.

According to the present invention the compositions as described above are particularly useful for treating cancer in a patient in need thereof. For this, the compositions of the present invention may be administered directly to a tumor or to a wide variety of locations including, for example, into sites such as the cerebral spinal fluid, bone marrow, joints, arterial endothelial cells, rectum, buccal/sublingual, vagina, the lymph system, to an organ selected from the group consisting of lung, liver, spleen, skin, blood and brain, or to a site selected from the group consisting of tumors and interstitial spaces. Within other embodiments, the composition may be administered intraocularly, intranasally, sublingually, orally, topically, intravesically, intrathecally, intravenously, intraperitoneally, intracranially, intramuscularly, intraarticularily or subcutaneously. Other representative routes of administration include gastroscopy, ECRP and colonoscopy, which do not require full operating procedures and hospitalization, but may require the presence of medical personnel.

Considerations for administering the compositions of the present invention include the following:

Oral administration is easy and convenient, economical (no sterility required), safe (over dosage can be treated in most cases), and permits controlled release of the active ingredient of the composition (the recombinant virus). Conversely, there may be local irritation such as nausea, vomiting or diarrhea, erratic absorption for poorly soluble drugs, and the recombinant virus will be subject to "first pass effect" by hepatic metabolism and gastric acid and enzymatic degradation. Further, there can be slow onset of action, efficient plasma levels may not be reached, a patient's cooperation is required, and food can affect absorption.

Buccal/sublingual administration is a convenient method of administration that provides rapid onset of action of the active component(s) of the composition, and avoids first pass metabolism. Thus, there is no gastric acid or enzymatic degradation, and the absorption of recombinant viruses is feasible. There is high bioavailability, and virtually immediate cessation of treatment is possible. Conversely, such administration is limited to relatively low dosages (typically about 10-15 mg), and there can be no simultaneous eating, drinking or swallowing.

Rectal administration provides a negligible first pass metabolism effect (there is a good blood/lymph vessel supply, and absorbed materials drain directly into the inferior vena cava), and the method is suitable for children, patients with emesis, and the unconscious. The method avoids gastric acid and enzymatic degradation, and the ionisation of a composition will not change because the rectal fluid has no buffer capacity (pH 6.8; charged compositions absorb best). Conversely, there may be slow, poor or erratic absorption, irritation, degradation by bacterial flora, and there is a small absorption surface (about 0.05 m²). Further, lipidophilic and water soluble compounds are preferred for absorption by the rectal mucosa, and absorption enhancers (e.g. salts, EDTA, NSAID) may be necessary.

Many of the routes of administration described herein (e.g., into the cerebrospinal fluid (CSF), into bone marrow, intraarticularily, intravenous, intraarterial, intracranial, intramuscular, subcutaneous, into various organs, intratumoral, into the interstitial spaces, intraperitoneal, intralymphatic, or into a capillary bed) may be accomplished simply by direct administration using a needle, catheter or related device. In particular, within certain embodiments of the invention, one or more dosages may be administered directly in the indicated manner:
into the cerebral spinal fluid at dosages greater than or equal to 10⁵, 10⁶, 10⁷, 10⁸, 10⁹, 10¹⁰, or 10¹¹ cfu ; into bone marrow at dosages greater than or equal to 10⁵, 10⁶, 10⁷, 10⁸, 10⁹, 10¹⁰, or 10" cfu; intraarticularily at dosages greater than or equal to 10⁵, 10⁶, 10⁷, 10⁸, 10⁹, 10¹⁰, or 10¹¹ cfu; intravenously at dosages greater than or equal to 10⁸, 10⁹, 10¹⁰, or 10¹¹ cfu; intraarterially at dosages greater than or equal to 10⁵, 10⁶, 10⁷, 10⁸, 10⁹, 10¹⁰, or 10¹¹ cfu; intracranially at dosages greater than or equal to 10⁹, 10¹⁰, or 10¹¹ cfu; intramuscularly at dosages greater than or equal to 10¹⁰, or 10¹¹ cfu;
intraocularily at dosages greater than or equal to 10⁵, 10⁶, 10⁷, 10⁸, 10⁹, 10¹⁰, or 10¹¹ cfu; pulmonarily at dosages greater than or equal to 10⁵, 10⁶, 10⁷, 10⁸, 10⁹, 10¹⁰, or 10¹¹ cfu; nasally at dosages greater than or equal to 10⁵, 10⁶, 10⁷, 10⁸, 10⁹, 10¹⁰, or 10¹¹ cfu; sublingually at dosages greater than or equal to 10⁵, 10⁶, 10⁷, 10⁸, 10⁹, 10¹⁰, or 10¹¹ cfu; rectally at dosages greater than or equal to 10⁵, 10⁶, 10⁷, 10⁸, 10⁹, 10¹⁰, or 10¹¹ cfu; orally at dosages greater than or equal to 10⁵, 10⁶, 10⁷, 10⁸, 10 ⁹,10¹⁰, or 10¹¹ cfu; topically at dosages greater than or equal to 10⁵, 10⁶, 10⁷, 10⁸, 10⁹, 10¹⁰, or 10¹¹ cfu; vaginally at dosages greater than or equal to 10⁵, 10⁶, 10⁷, 10⁸, 10⁹, 10¹⁰, or 10¹¹ cfu; subcutaneously at dosages greater than or equal to 10⁹, 10¹⁰, or 10¹¹ cfu;
intervesically at dosages greater than or equal to 10⁵, 10⁶, 10⁷, 10⁸, 10⁹, 10¹⁰, or 10¹¹ cfu; into an organ such as the lung, liver, spleen, skin, blood or brain at dosages greater than or equal to 10⁵, 10⁶, 10⁷, 10⁸, 10⁹, 10¹⁰, or 10¹¹ cfu;
intratumour at dosages greater than or equal to 10⁸, 10⁹, 10¹⁰, or 10¹¹ cfu;
intraperitoneally at dosages greater than or equal to 10⁸, 10⁹, 10¹⁰, or 10¹¹ cfu;
interstitial spaces at dosages greater than or equal to 10¹⁰ or 10¹¹ cfu;
intralymphatically at dosages greater than or equal to 10⁵, 10⁶, 10⁷, 10⁸, 10⁹, 10¹⁰, or 10¹¹ cfu; into a capillary bed at dosages greater than or equal to 10⁵, 10⁶, 10⁷, 10⁸, 10⁹, 10¹⁰, or 10¹¹ cfu; or intrathecally at dosages greater than or equal to 10⁵, 10⁶, 10⁷, 10⁸, 10⁹, 10¹⁰, or 10¹¹ cfu.

Recombinant virus may be delivered to the target from outside of the body (as an outpatient procedure) or as a surgical procedure, where the vector is administered as part of a procedure with other purposes, or as a procedure designed expressly to administer the vector. Other routes and methods for administration include the non-parenteral routes as well as administration via multiple sites.

### SHORT DESCRIPTION OF THE DRAWINGS

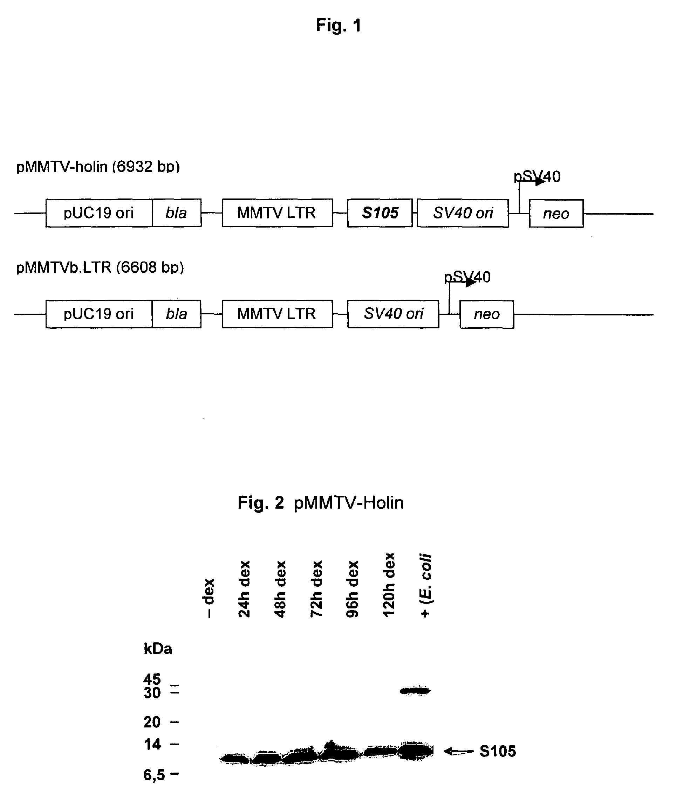

**Fig. 1** shows a schematical drawing of the expression vectors pMMTV-holin and pMMTVb.LTR: bla, β-lactamase gene; LTR, 3' LTR of Mouse Mammary Tumor Virus (MMTV); *S105,* bacteriophage λ gene S (codons 3 to 107); SV40 ori, SV40 origin of replication; pSV40, SV40 promoter; neo, neomycin resistance gene.
**Fig. 2** shows the expression of *S105* in HeLa cells transfected with pMMTV-holin after stimulation with dexamethasone for the indicated times as determined by Western blotting. The S105 band is indicated by an arrow. The sizes of the molecular weight standard are given on the left. - dex, no incubation with dexamethasone; + *(E. coli),* lysate of *E. coli* cells expressing *S105.*

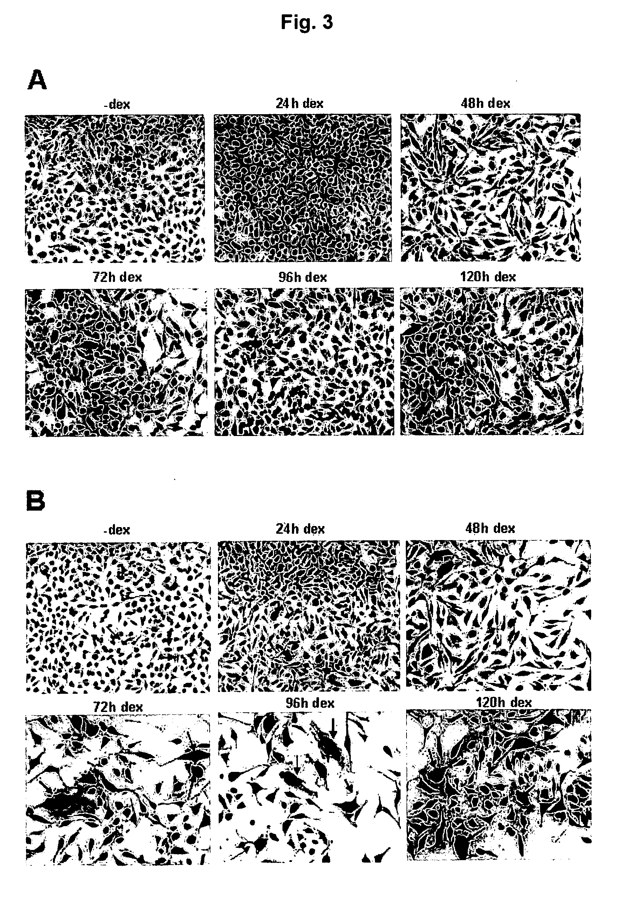

**Fig. 3** shows the morphological changes of HeLa cells transfected with pMMTV-holin after dexamethasone-induced *S105* expression. A. HeLa cells stably transfected with pMMTVb.LTR and incubated with dexamethasone for the indicated times. B. HeLa cells stably transfected with pMMTV-holin and incubated with dexamethasone (dex) for the indicated times. - dex, no incubation with dexamethasone. Arrows point to nuclei of multinucleic cells.

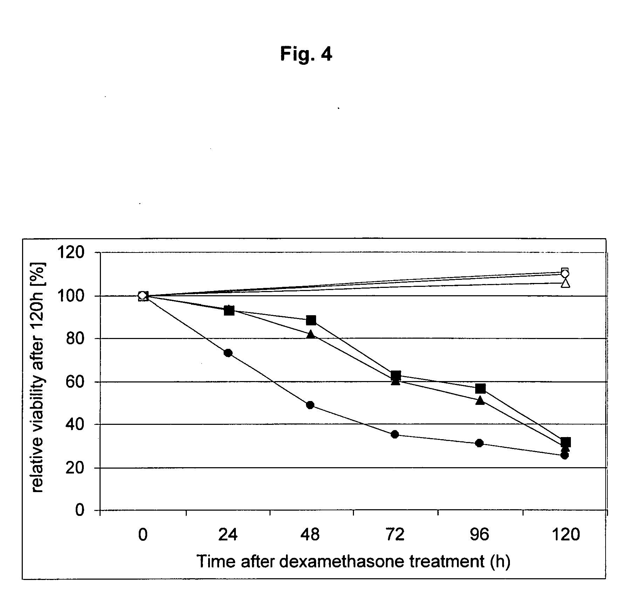

**Fig. 4** shows the effect of dexamethasone-induced S105 expression in HeLa/pMMTV-holin cells on the cellular metabolism as determined by the measurement of relative NADH and NADPH levels using the MTT test in (•) HeLa cells stably transfected with pMMTV-holin and in (O) HeLa cells stably transfected with pMMTVb.LTR as a negative control, and on the membrane integrity as determined by trypan blue staining in (▲) HeLa cells stably transfected with pMMTV-holin and in (Δ) HeLa cells stably transfected with pMMTVb.LTR as a negative control, and on the cytoplasmic membrane as determined by annexinV/7AAD staining in (■) HeLa cells stably transfected with pMMTV-holin and in (□) HeLa cells stably transfected with pMMTVb.LTR as a control.

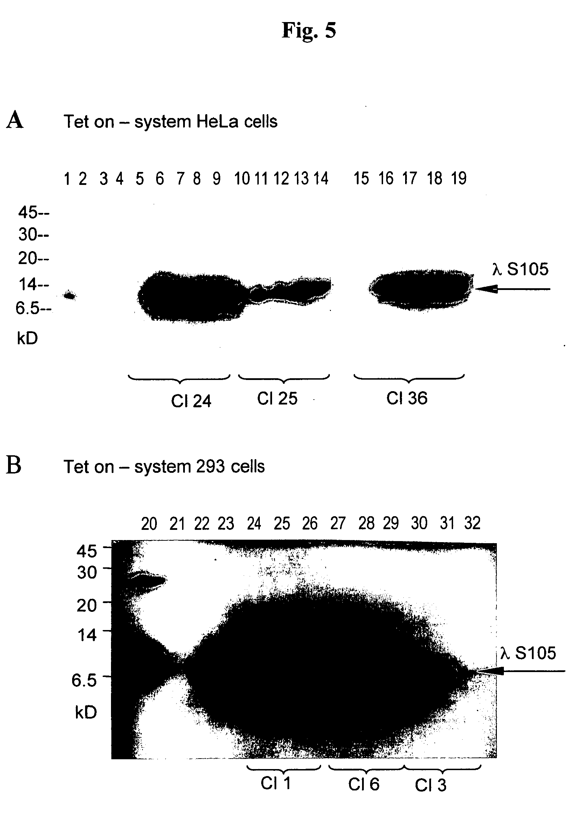

**Fig. 5** shows the expression of *S105* in (A) HeLa cell clone Cl24 (lanes 5-9), clone CI35 (lanes 10-15), clone CI36 (lanes 15-19) and (B) 293 cell clone CI1 (lanes 24-26), clone CI6 (lanes 27-29), clone CI3 (lanes 30-32) stably transfected with a S105- containing Tet-On expression vector after stimulation with doxycyclin as determined by Western blotting. The *S105* band is indicated by an arrow. The sizes of the protein molecular weight standard are given on the left.
   Numbering of lanes are (1, 20) lysates of E. coli expressing holin, (2, 23) extracts of HeLa cells stably transfected with pMMTV and treated with dexamethasone as a negative control, (3, 21) protein standard, (4, 22) extracts of HeLa cells stably transfected with pMMTV-holin, (5, 10, 15, 25, 27, 30) cells and incubated without doxycyclin, cells incubated with doxycyclin for 24h (6, 11, 16, 26, 28, 31), 48h (7, 12, 17, 24, 29, 32), 72h (8, 13, 18), and 96h (9, 14, 19)
**Fig. 6** shows the effect of doxycyclin-induced S105 expression in (A) HeLa and (B) in 293 cell clones stably transfected with a S105-containing Tet-On expression vector on the cellular metabolism as determined by the measurement of relative NADH and
   N A D P H .
   A. ◆, HeLa neg. ctrl. cells; ■, HeLa clone 24; **▲**, HeLa clone 35; *, HeLa clone 36.
   B. ◆, 293 neg. ctrl. cells; ■, 293 clone 3; A, 293 clone 6; *, 293 clone 1.
**Fig. 7** shows the effect of doxycyclin-induced S105 expression on the cytoplasmic membrane of (A) HeLa and (B) 293 cell clones stably transfected with a S105- containing Tet-On expression vector as determined by trypan blue staining.
   A. ◆, HeLa neg. ctrl. cells; ■, HeLa clone 24; **▲**, HeLa clone 35; *, HeLa clone 36.
   B. ◆, 293 neg. ctrl. cells; ■, 293 clone 3; ▲, 293 clone 6; *, 293 clone 1.

The following examples serve to illustrate certain aspects of the present invention.

### EXAMPLES

### Example 1

### Functionality of a vector expressing the phage Lambda (λ) S105 protein

In this example, the functionality of the vector used to express S105 in HeLa cells is examined. The part of the S^{λ} ORF encoding the S105 allele (codons 3 to 107; nucleotides 45192 to 45509 of the λ genome [GenBank accession number NC001416]) was amplified by PCR from plasmid pVIII-S105 [53] using primers V13 and W13 (Table 1), restricted with Xbal and Sa*c*ll and ligated with the vector pMMTVGFP (Table 1) that had also been restricted with Xbal and Sacll, resulting in vector pMMTV-holin (Fig. 1). pMMTVGFP, the parental vector of pMMTV-holin was constructed as follows: the CMV promoter-containing *Nru*l-*8am*Hl fragment of pcDNA3 (Table 1) was replaced with a MMTV-LTR (mouse mammary tumor virus long terminal repeat)-containing *Nru*l*-Bam*Hl fragment of vector pLTR.stp (Table 1), resulting in vector pMMTVb.LTR (Table 1). The eGFP gene was cut out of pEGFP-1 (Table 1) as an EcoRl-Notl fragment and ligated with pMMTVb.LTR that had been restricted with EcoRl and *Not*l, giving rise to pMMTVGFP.

*E.coli* DH10B cells (Invitrogen) were transformed with pMMTV-holin and ampicillin-resistant clones were isolated. Correct construction of the vector pMMTV-holin was confirmed by restriction enzyme analysis of prepared plasmid DNA and the integrity of the S105 gene was proven by DNA sequencing using the primer Seq-RC-MMTV-1-R (Table 1).

HeLa cells were stably transfected with pMMTV-holin using the calcium phosphate method according to the instructions of the manufacturer (Invitrogen) and individual clones resistant to Geneticin G418 (Invitrogen) were analyzed for S105 expression by Western blotting using an antiserum raised against a peptide located in the C-terminal part of S105. pMMTV-holin allows glucocorticoid-inducible expression of S105 from the Mouse Mammary Tumor Virus (MMTV) promoter located in the U3 region of the MMTV 3' LTR. For screening of S*105*-positive cells, expression was induced by addition of 1 µM dexamethasone (Sigma) to the culture medium 48 h prior to harvesting of the cells. One positive clone (clone number 12) was further investigated. Fig. 2 shows the expression of S105 in clone number 12 after induction with dexamethasone for different time periods.

1x10⁵ HeLa cells stably transfected with pMMTV-holin or, as a control with pMMTVb.LTR, were seeded into 6-well-plates and cultivated for 120 h. At different time points, 1 µM dexamethasone was added to the culture medium and the cells were further incubated for an additional 24, 48, 72, 96, and 120 h in the presence of the hormone. For cells, that were treated with dexamethasone for more than 48 h, fresh dexamethasone was added to the culture medium every second day. At time point 120 h, all cells were harvested simultaneously, resuspended in SDS-sample buffer (Laemmli) and equal amounts of protein were analyzed by Western blotting using standard techniques. *S105* expression could first be detected after 24 hours of incubation with dexamethasone. Expression levels stayed more or less constant for the rest of the experiment (Fig. 2). As a positive control, a cell lysate of *E. coli* cells expressing *S105* was used (11).

The integrity of the *S105* gene integrated into chromosomal DNA of stably transfected HeLa cells was confirmed by amplifying the coding sequence by PCR using the primers Holin-F2 and Holin-R1 (Table 1), both binding upstream and downstream, respectively, of the S105 open reading frame (ORF) and subsequent sequencing of the fragment with the primers Holin-F2 and Holin-R2 (Table 1).

### EXAMPLE 2

### Morphological changes of HeLa cells after dexamethasone-induced S105 expression

In this example, the morphological changes of HeLa cells after dexamethasone-induced *S105* expression are examined. Briefly, 1x10⁴ HeLa cells stably transfected with pMMTV-holin or, as a control, pMMTVb.LTR were seeded into the chambers of 8-well chamber slides and cultivated for 120 h. At different defined time points, 1 µM dexamethasone was added to the culture medium and the cells were further incubated for an additional 24, 48, 72, 96, and 120 h in the presence of the hormone. For cells that were treated with dexamethasone for more than 48 h, fresh dexamethasone was added to the culture medium every second day. Cells were fixed in buffered formalin and and counterstained for 2 min in hemalaun solution (Fluka). Slides were analyzed by standard light microscopy (Zeiss Axiovert 200) and photographed. While cells not expressing *S105* did not show any significant change in their morphology after dexamethasone treatment (Fig. 3A), dexamethasone-induced expression of *S105* in HeLa cells with time provoked a kind of "swelling", eventually resulting in huge, often multinucleic cells (Fig. 3B; arrows point to the nuclei of multinucleic cells). In addition, according to the findings described in example 1, in comparison to the respective controls stably transfected with pMMTVb.LTR (Fig. 3A) cell numbers decreased in pMMTV-holin transfected cells starting 48 h after induction of S105 expression (Fig. 3B, 48 h dex, 72 h dex, 96 h dex, 120 h dex).

### EXAMPLE 3

### Effect of dexamethasone-induced S105 expression on cellular metabolism

In this example, the effect of dexamethasone-induced *S105* expression in HeLa cells on cellular metabolism as determined by the measurement of relative NADH and NADPH levels is described.

Briefly, 1×10⁵ HeLa cells stably transfected with pMMTV-holin or, as a negative control, pMMTVb.LTR were seeded into 6-well-plates and cultivated for 120 h. At different defined time points, 1 µM dexamethasone was added to the culture medium and the cells were further incubated for an additional 24, 48, 72, 96, and 120 h in the presence of the hormone. For cells that were treated with dexamethasone for more than 48 h, fresh dexamethasone was added to the culture medium every second day. At time point 120 h, all cells were harvested simultaneously and subjected to a MTT assay (Sigma) for determining the relative amount of NADH and NADPH as an indicator for cell metabolism which, in turn, is an indicator for cell viability. For HeLa cells stably transfected with pMMTV-holin, incubation with dexamethasone for 120 h resulted in a significant decrease of viable cells by 74% when compared to cells that were not treated with dexamethasone (Fig. 4, empty circles). In order to exclude a negative effect of dexamethasone itself on cell growth and viability, HeLa cells stably transfected with pMMTVb.LTR lacking the *S105* encoding region were also incubated with dexamethasone for 120 h. When viable cell numbers of HeLa cells stably transfected with pMMTV-holin and grown for 120 h but not incubated with dexamethasone (filled circle at time point 0 h in Fig. 4) are set to 100% and compared to HeLa cells stably transfected with pMMTVb.LTR but otherwise treated in the same way (empty circle at time point 120 h in Fig. 4), it is obvious, that dexamethasone had no negative effect on cell growth. In contrast, HeLa cells lacking the *S105* gene revealed a higher number of viable cells (110%) when compared to cells harboring the gene (100%). This effect can be explained by the leakiness of the MMTV promoter system and a constitutive low level expression of *S105* in cells transfected with pMMTV-holin even in the absence of dexamethasone or due to low levels of glucocorticoids present in the cell culture medium. Taken together, these results demonstrate that S105 expression has a pronounced negative effect on cell viability as indicated by a shut-down of cellular metabolism.

### EXAMPLE 4

### Effect of dexamethasone-induced S105 expression on membrane integrity

In this example, the effect of dexamethasone-induced *S105* expression in HeLa cells on membrane integrity as determined by trypan blue staining is described. Leakiness of the cytoplasmic membrane is a well-known indicator for apoptosis or necrosis.

1x10⁵ HeLa cells stably transfected with pMMTV-holin or, as a control, pMMTVb.LTR were seeded into 6-well-plates and cultivated for 120 h. At different defined time points, 1 µM dexamethasone was added to the culture medium and the cells were further incubated for an additional 24, 48, 72, 96, and 120 h in the presence of the hormone. For cells, that were treated with dexamethasone for more than 48 h, fresh dexamethasone was added to the culture medium every second day. At time point 120 h, all cells were harvested simultaneously and subjected to trypan blue staining as an indicator for membrane integrity. Trypan blue staining was performed according to the instructions of the manufacturer (Sigma). For HeLa cells stably transfected with pMMTV-holin, incubation with dexamethasone for 120 h resulted in a significant lower number of cells with an intact (trypan blue-excluding) membrane (30%) when compared to cells that were not treated with dexamethasone and that were set to 100% (Fig. 4, filled triangles). In order to exclude a negative effect of dexamethasone itself on membrane integrity, HeLa cells stably transfected with pMMTVb.LTR lacking S105 were also incubated with dexamethasone for 120 h. When the numbers of non-compromised HeLa cells stably transfected with pMMTV-holin that had been grown for 120 h but had not been incubated with dexamethasone (100%; filled triangle at time point 0 h in Fig. 4) are compared to HeLa cells stably transfected with pMMTVb.LTR but otherwise treated in the same way (empty triangle at time point 120 h in Fig. 4), it is obvious, that dexamethasone itself did not cause the membrane damage. In contrast, HeLa cells lacking the *S105* gene revealed a higher number of cells with an intact cytoplasmic membrane (106%) when compared to cells harboring the gene (100%). As also mentioned in example 3, this effect is most likely due to the leakiness of the MMTV promoter system. Taken together, the results described in this example demonstrate the damaging effect of S105 protein on the cytoplasmic membrane.

### EXAMPLE 5

### Effect of dexamethasone-induced S105 expression on the cytoplasmic membrane

In this example, the effect of dexamethasone-induced expression of protein S*105* on the cytoplasmic membrane as determined by annexin V-PE/7AAD staining is described. Annexin V is the natural binding partner for phosphatidylserine (PS) located in membrane leaflets which face the cytosol. However, during apoptosis of cells, PS is exposed at the cell surface and can be detected by annexin V added from outside. Binding of annexin V to cells therefore is an indicator for apoptosis but also necrosis since cells with compromised membranes will allow annexin V to enter and bind PS from inside (55). 7AAD (7-amino-actinomycin D) intercalates into double-stranded nucleic acids. It is excluded by viable cells but can penetrate cell membranes of late apoptotic and/or necrotic cells.

1x10⁵ HeLa cells stably transfected with pMMTV-holin or pMMTVb.LTR lacking the S105 encoding region were seeded into 6-well-plates and cultivated for 120 h. At different defined time points, 1 µM dexamethasone was added to the culture medium and the cells were further incubated for an additional 24, 48, 72, 96, and 120 h in the presence of the hormone. For cells, that were treated with dexamethasone for more than 48 h, fresh dexamethasone was added to the culture medium every second day. At time point 120 h, all cells were harvested simultaneously and subjected to annexinV-PE/7AAD staining according to the instructions of the manufacturer (BD Biosciences).

When the number of cells that had not been treated with dexamethasone and that were negative for annexin V-PE/7AAD staining (therefore regarded as living cells) are set to 100% and compared to the number of annexin V-PE/7AAD-negative cells of the pool of cells that had been treated with dexamethasone for 120 h, it becomes obvious that much less cells (32%) are viable (Fig. 4). In order to investigate a possible effect of dexamethasone itself on membrane integrity, HeLa cells stably transfected with pMMTVb.LTR lacking the *S105* gene were also incubated with dexamethasone for 120 h. When the numbers of non-compromised HeLa cells stably transfected with pMMTV-holin that had been grown for 120 h but had not been incubated with dexamethasone (100%; filled squares at time point 0 h in Fig. 4) are compared to non-compromised HeLa cells stably transfected with pMMTVb.LTR but otherwise treated in the same way (empty squares at time point 120 h in Fig. 4), it is obvious, that dexamethasone itself did not cause the membrane damage. As also pointed out in examples 3 and 4 HeLa cells lacking *S105* revealed a higher number of viable cells after 120 h of dexamethasone treatment (111%) when compared to cells harboring the gene (100%). Again, this effect is most likely due to the leakiness of the MMTV promoter system (see example 3). Taken together, this example demonstrates the powerful cell killing effect of S105 when expressed in HeLa cells.

### Example 6

### Evaluation of a doxycyclin-induced vector system

In this example, the effect of S105 expression in HeLa and 293 cells using a doxycyclin-inducible Tet-On gene expression system (BD Biosciences) is described.

Stably transfected cell clones were analyzed for doxycyclin-inducible *S105* expression by Western blotting using an antiserum raised against a peptide located in the C-terminal part of *S105.* Among the *S105* expressing clones, the three clones showing highest expression were further analyzed. HeLa cells were cultivated in the presence of 5 µg/ml doxycyclin for 24, 48, 72, and 96 h. 293 cells were cultivated only for 24 and 48 h since all cells were dead after this incubation period. S105 expression in HeLa cell clones 24, 35, 36 and in 293 cell clones 1, 3, and 6 is shown in Fig. 5. While no (Fig. 5, lanes 5, 15, 27, 30) or low (Fig. 5, lanes 10, 25) background expression was observed in HeLa and 293 control cells that had not been incubated with doxycyclin, very high expression was observed already 24h after addition of doxycyclin to the culture medium (Fig. 5, 6, 11, 16, 26, 28, 31). As positive controls, the same cell lysate of E. *coli* cells expressing *S105* as described in example 1 as well as a cell extract of HeLa cells stably expressing S*105* from vector pMMTV-holin (see example 1) were used. Compared to HeLa cells expressing S105 from vector pMMTV-holin (fig. 5, lane 22), the expression rate could dramatically be enhanced by expressing S*105* with the Tet-On vector. The low amount of S105 detected in extracts of 293 clone 3 after 48 h of incubation with doxycyclin by Western blotting (Fig. 5, lane 32) is due to the fact that only a low amount of protein could be loaded because of the few cells remained after this treatment. The higher band visible in the positive control (Fig. 5, lane 20) most probably represents an oligomeric form of protein *S105* while the bands in Fig. 5B running lower than the bands of the holin protein (marked by an arrow) may indicate partial degradation of protein S105 in 293 cells.

### Example 7

### Effect of doxycyclin-induced S105 expression on cellular metabolism

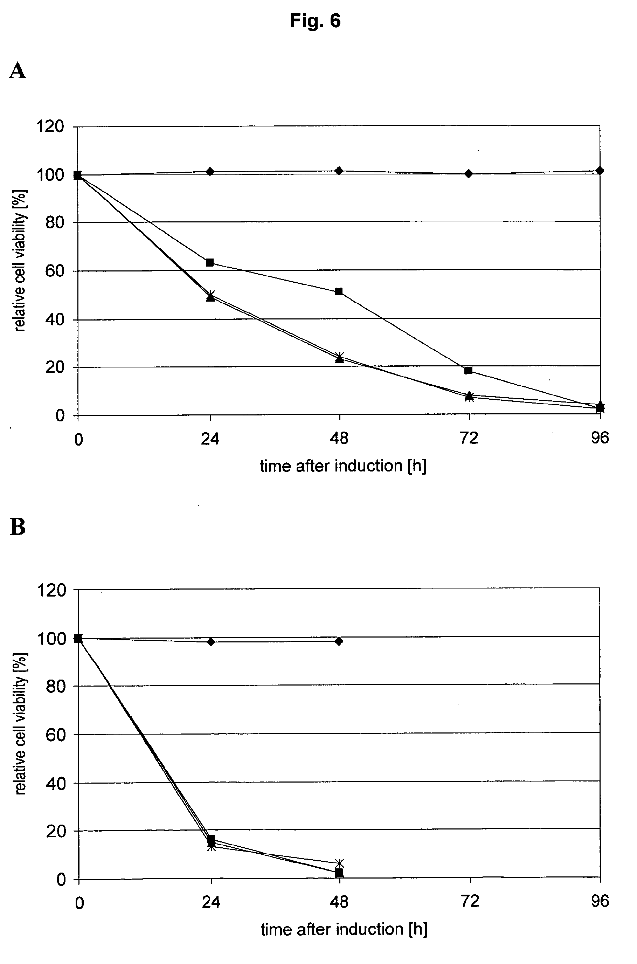

This example describes the effect of doxycyclin-induced S105 expression in HeLa and 293 cells on the cellular metabolism as determined by the measurement of relative NADH and NADPH levels as an indicator for cell viability. S105 was expressed using a Tet-On expression system (BD Biosciences). HeLa cell clones 24, 35, 36 and 293 cell clones 1, 3, and 6 harbouring the S105-containing Tet-On gene expression vector were cultivated in the presence of 5 µg/ml doxycyclin for 24, 48, 72, and 96 h (HeLa cell clones) or 24 and 48 h (293 cell clones) to induce S105 expression. S105 expression was verified by Western blotting (see example 6). Cells were subjected to a MTT assay (see example 3) for determining cell viabilities (Fig. 6). Viabilities of HeLa and 293 control cells lacking the vector were not impaired indicating that doxycyclin alone had no negative effect on cell viabilities. However, expression of S105 in HeLa and 293 cells respectively, containing the expression vector resulted in a dramatic cell killing rate of 94% to 98% after 96 hours of incubation with doxycyclin.

### Example 8

### Effect of doxycyclin-induced S105 espression on cytoplasmic membrane integrity

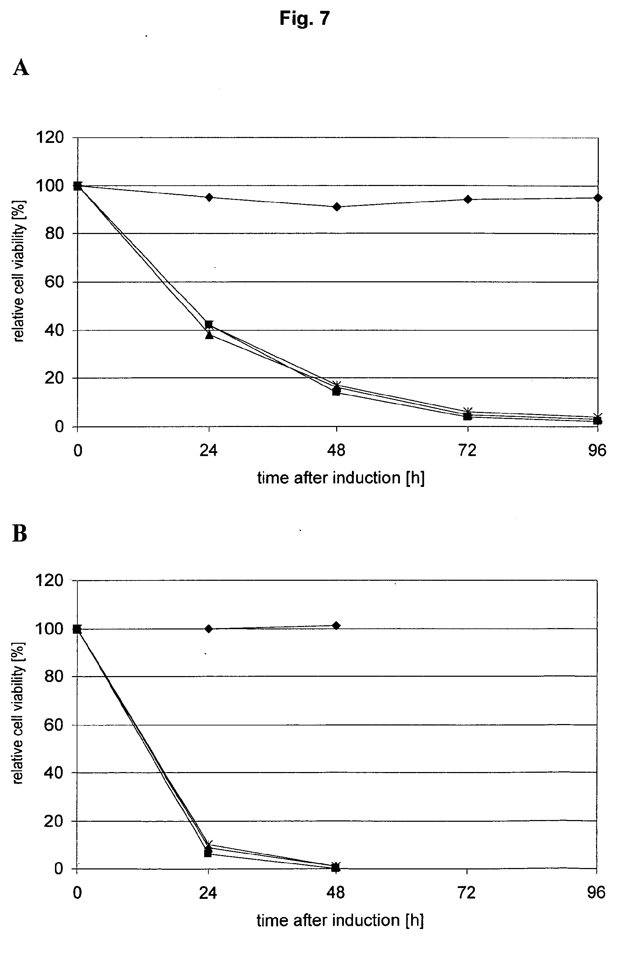

In this example, the effect of doxycyclin-induced *S105* expression in HeLa and 293 cells on the cytoplasmic membrane integrity as an indicator of cell viability as determined by trypan blue staining is described. S105 protein was expressed using a doxycyclin-inducible Tet-On gene expression system (BD Biosciences). HeLa cell clones 24, 35, 36 and 293 cell clones 1, 3, and 6 harbouring the S105-containing Tet-On gene expression vector were examined. S105 expression was induced by adding 5 µM doxycyclin and cells were cultivated in the presence of doxycyclin for 24, 48, 72, and 96 h (HeLa cell clones) or 24 and 48 h (293 cell clones). *S105* protein expression was verified by Western blotting (see example 6). Trypan blue staining (see example 4) was performed according to the instructions of the manufacturer (Sigma). For HeLa and 293 cells stably transfected with the S105-containing Tet-On vector, incubation with doxycyclin for 96 h resulted in a killing rate of 97% to 100% whereas HeLa and 293 control cells lacking the vector showed no impairment of their membrane integrity (Fig. 7). This obvious damage of the cytoplasmic membrane may either be due to apoptosis or necrosis mediated by S105.

### REFERENCES CITED

1. Aghi, M., F. Hochberg, and X.O. Breakefield, *Prodrug activation enzymes in cancer gene therapy.* J Gene Med, 2000. 2(3): p. 148-64.
2. Moolten, F.L., *Tumor chemosensitivity conferred by inserted herpes thymidine kinase genes: paradigm for a prospective cancer control strategy.* Cancer Res, 1986. **46**(10): p. 5276-81.
3. Danielsen, S., *et al*., *Characterization of the Escherichia coli codBA operon encoding cytosine permease and cytosine deaminase.* Mol Microbiol, 1992. **6**(10): p. 1335-44.
4. Weber, G.F. and D.J. Waxman, *Activation of the anti-cancer drug ifosphamide by rat liver microsomal P450 enzymes.* Biochem Pharmacol, 1993. **45**(8): p. 1685-94.
5. Wei, M.X., *et al., Experimental tumor therapy in mice using the cyclophosphamide-activating cytochrome P450 2B1 gene.* Hum Gene Ther, 1994. 5(8): p. 969-78.
6. Rigg, A. and K. Sikora, *Genetic prodrug activation therapy.* Mol Med Today, 1997. 3(8): p. 359-66.
7. Igney, F.H. and P.H. Krammer, *Death and anti-death: tumour resistance to apoptosis.* Nat Rev Cancer, 2002. 2(4): p. 277-88.
8. Schwartz, P.S. and D.J. Waxman, *Cyclophosphamide induces caspase 9-dependent apoptosis in 9L tumor cells.* Mol Pharmacol, 2001. **60**(6): p. 1268-79.
9. Diaz, R.M., *et al., A lentiviral vector expressing a fusogenic glycoprotein for cancer gene therapy.* Gene Ther, 2000. 7(19): p. 1656-63.
10. Fielding, A.K., *et al., A hyperfusogenic gibbon ape leukemia envelope glycoprotein: targeting of* a *cytotoxic gene by ligand display.* Hum Gene Ther, 2000. **11**(6): p. 817-26.
11. Galanis, E., *et al., Use of viral fusogenic membrane glycoproteins* as *novel therapeutic transgenes in gliomas.* Hum Gene Ther, 2001. **12**(7): p. 811-21.
12. Bateman, A., *et al., Fusogenic membrane glycoproteins* as a *novel class of genes for the local and immune-mediated control of tumor growth.* Cancer Res, 2000. **60**(6): p. 1492-7.
13. Bateman, A.R., *et al., Viral fusogenic membrane glycoproteins kill solid tumor cells by nonapoptotic mechanisms that promote cross presentation of tumor antigens by dendritic* cells. Cancer Res, 2002. **62**(22): p. 6566-78.
14. Higuchi, H., *et al., Viral fusogenic membrane glycoprotein expression* causes *syncytia formation with bioenergetic cell death: implications for gene therapy.* Cancer Res, 2000. 60(22): p. 6396-402.
15. Lidor, Y.J., *et al., In vitro expression* of *the diphtheria toxin A-chain gene under the control* of *human chorionic gonadotropin gene promoters as a means of directing toxicity to ovarian cancer cell lines.* Am J Obstet Gynecol, 1997. **177**(3): p. 579-85.
16. Massuda, E.S., *et al., Regulated expression* of *the diphtheria toxin A chain by* a *tumor-specific chimeric transcription factor results in selective toxicity for alveolar rhabdomyosarcoma cells.* Proc Natl Acad Sci U S A, 1997. **94**(26): p. 14701-6.
17. Maxwell, I.H., L.M. Glode, and F. Maxwell, *Expression* of *diphtheria toxin A-chain in mature B-cells:* a *potential approach to therapy* of *B-lymphoid malignancy.* Leuk Lymphoma, 1992. 7(5-6): p. 457-62.
18. Maxwell, I.H., F. Maxwell, and L.M. Glode, *Regulated expression* of a *diphtheria toxin* A-*chain gene transfected into human* cells: *possible strategy for inducing cancer cell suicide.* Cancer Res, 1986. **46**(9): p. 4660-4.
19. Allam, M., *et al., Cholera toxin triggers apoptosis in human lung* cancer *cell lines.* Cancer Res, 1997. 57(13): p. 2615-8.
20. Kiura, K., *et al., Inhibitory effects* of *cholera toxin on in vitro growth* of *human lung* cancer *cell lines.* Anticancer Drug Des, 1993. **8**(6): p. 417-28.
21. Bodart, J.F., *et al., Anthrax, MEK and* cancer Cell Cycle, 2002. **1**(1): p. 10-5.
22. Frankel, A.E., *et al., Anthrax fusion protein therapy* of cancer. Curr Protein Pept Sci, 2002. 3(4): p. 399-407.
23. Liu, S., T.H. Bugge, and S.H. Leppla, *Targeting* of *tumor* cells *by cell surface urokinase plasminogen activator-dependent anthrax toxin.* J Biol Chem, 2001. **276**(21): p. 17976-84.
24. Liu, S., *et al., Potent antitumor activity* of a *urokinase-activated engineered anthrax toxin.* Proc Natl Acad Sci U S A, 2003. **100**(2): p. 657-62.
25. Weber, E., W.F. Anderson, and N. Kasahara, *Recent advances in retrovirus vector-mediated gene therapy: teaching an old vector new tricks.* Curr Opin Mol Ther, 2001. **3**(5): p. 439-53.
26. Cosset, F.L., *et al., High-titer packaging* cells *producing recombinant retroviruses resistant to human serum.* J Virol, 1995. **69**(12): p. 7430-6.
27. Saier, M.H., Jr., *Families* of *proteins forming transmembrane channels.* J Membr Biol, 2000. 175(3): p. 165-80.
28. Young, R. and U. Bläsi, *Holins: form and function in bacteriophage lysis.* FEMS Microbiol Rev, 1995. 17(1-2): p. 191-205.
29. Wang, I.N., D.L. Smith, and R. Young, *Holins: the protein* clocks *of bacteriophage infections.* Annu Rev Microbiol, 2000. **54:** p. 799-825.
30. Barenboim, M., et *al.*, *Characterization of the dual start motif of* a class *II holin gene.* Mol Microbiol, 1999. 32(4): p. 715-27.
31. Bläsi, U., *et al., The C-terminal sequence of the lambda holin constitutes* a *cytoplasmic regulatory domain.* J Bacteriol, 1999. **181**(9): p. 2922-9.
32. Zagotta, M.T. and D.B. Wilson, *Oligomerization of the bacteriophage lambda* S *protein in the inner* membrane *of Escherichia coli.* J Bacteriol, 1990. **172**(2): p. 912-21.
33. Smith, D.L., *et al*., *Purification and biochemical characterization of the lambda holin.* J Bacteriol, 1998. 180(9): p. 2531-40.
34. Chang, C.Y., K. Nam, and R. Young, S gene *expression and the timing of lysis by bacteriophage lambda.* J Bacteriol, 1995. **177**(11): p. 3283-94.
35. Bläsi, U. and R. Young, *Two beginnings for* a *single purpose: the dual-start holins in the regulation of phage lysis.* Mol Microbiol, 1996. **21** (4): p. 675-82.
36. Steiner, M., W. Lubitz, and U. Bläsi, The *missing link in phage lysis of gram-positive bacteria: gene* 14 *of Bacillus subtilis phage phi* 29 *encodes the functional homolog of* lambda S *protein.* J Bacteriol, 1993. **175**(4): p. 1038-42.
37. Tedin, K., *et al., Dual translational* start *motif evolutionarily* conserved *in the holin gene of Bacillus subtilis phage phi* 29. Virology, 1995. **206**(1): p. 479-84.
38. Grundling, A., *et al., Dimerization* between *the holin and holin inhibitor* of phage *lambda.* J Bacteriol, 2000. **182**(21): p. 6075-81.
39. Wang, I.N., J. Deaton, and R. Young, *Sizing the holin lesion with an endolysin-beta-*galactosidase *fusion.* J Bacteriol, 2003. **185**(3): p. 779-87.
40. Young, K.D. and R. Young, *Lytic action of cloned phi X174* gene E. J Virol, 1982. **44**(3): p. 993-1002.
41. Henrich, B., W. Lubitz, and R. Plapp, *Lysis of Escherichia coli by induction of cloned phi* X174 genes. Mol Gen Genet, 1982. **185**(3): p. 493-7.
42. Winter, R.B. and L. Gold, *Overproduction of bacteriophage* Q *beta maturation (A2) protein* leads *to cell lysis.* Cell, 1983. **33**(3): p. 877-85.
43. Witte, A., *et al.*, *Mutations in cell division proteins FtsZ and FtsA inhibit phiX174 protein-E-mediated lysis of Escherichia coli.* Arch Microbiol, 1998. **170**(4): p. 259-68.
44. Witte, A., *et al*., *Phi X174 protein E-mediated lysis of Escherichia coli.* Biochimie, 1990. 72(2-3): p. 191-200.
45. Roof, W.D., *et al., slyD,* a *host* gene *required for phi X174 lysis, is related to the* FK506-*binding protein family of peptidyl-prolyl cis-trans-isomerases.* J Biol Chem, 1994. **269**(4): p. 2902-10.
46. Bernhardt, T.G., W.D. Roof, and R. Young, *The Escherichia coli FKBP-type PPlase SlyD is required for the stabilization of the E lysis protein of bacteriophage phi X174.* Mol Microbiol, 2002.45(1): p. 99-108.
47. Bernhardt, T.G., W.D. Roof, and R. Young, *Genetic evidence that the bacteriophage phi X174 lysis protein inhibits cell wall synthesis.* Proc Natl Acad Sci U S A, 2000. 97(8): p. 4297-302.
48. Bernhardt, T.G., D.K. Struck, and R. Young, *The lysis protein E of phi X174 is* a *specific inhibitor of the MraY- catalyzed step in peptidoglycan synthesis.* J Biol Chem, 2001. **276**(9): p. 6093-7.
49. Bernhardt, T.G., *et al.,* A *protein antibiotic in the phage Qbeta virion: diversity in lysis* targets. Science, 2001. **292**(5525): p. 2326-9.
50. Witte, A., *et al.*, *Endogenous transmembrane tunnel formation mediated by phi X174 lysis protein E.* J Bacteriol, 1990. **172**(7): p. 4109-14.
51. Garrett, J., C. Bruno, and R. Young, *Lysis protein* S *of phage lambda functions in Saccharomyces cerevisiae.* J Bacteriol, 1990. **172**(12): p. 7275-7.
52. Tabotta, W., *et al., Genetic reshuffling reconstitutes functional expression cassettes in retroviral vectors.* J Gene Med, 2001. **3**(5): p. 418-26.
53. Graschopf, A. and U. Bläsi, *Molecular function of the dual-start motif in the lambda* S *holin.* Mol Microbiol, 1999. **33**(3): p. 569-82.
54. Bläsi, U., *et al*., *Dual translational initiation sites control function of the lambda* S *gene.* Embo J, 1989. **8**(11): p. 3501-10.
55. Koopman G., *et al., Annexin V for flow cytometric* detection *of phosphatidylserine expression on B cells undergoing apoptosis.* Blood. 1994. **84**(5): p.1415-20
56. Plumb JA, Bilsland A, Kakani R, Zhao J, Glasspool RM, Knox RJ, Evans TRJ, Keith WN. Telomerase-specific suicide gene therapy vectors expressing bacterial nitroreductase sensitize human cancer cells to the pro-drug CB1954. Oncogene 2001. 20: p. 7797-7803
57. Cortes ML, de Felipe P, Martin V, Hughes MA, Izquierdo M: Successful use of a plant gene in the treatment of cancer *in vivo.* Gene *Ther* 1998, 5: p. 1499-1507
58. Martin, V., Cortes, M.L., de Felipe, P., Farsetti, A., Calcaterra, N.B., & Izquierdo, M. Cancer gene therapy by thyroid hormone-mediated expression of toxin genes *Cancer Res 2000,* **60**(12): p. 3218-3224
59. Kondo S, Barna BP, Morimura T, Takeuchi J, Yuan J, Akbasak A, Barnett GH: *Interleukin 1β-converting enzyme mediates cisplatin-induced apoptosis in malignant glioma cells.* Cancer Res. 1995, 55(24): p. 173-180
60. Kondo S, Tanaka Y, Kondo Y, Ishizaka Y, Hitomi M, Haqqi T, Liu J, Barnett GH, Alnemri ES, Barna BP: *Retroviral transfer of CPP32β gene into malignant gliomas in vitro and in vivo.* Cancer Res. 1998, **58**(5), p. 962-967
61. Kondo S, Ishizaka Y, Okada T, Kondo Y, Hitomi M, Tanaka Y, Haqqi T, Barnett GH, Barna BP: FADD *gene therapy for malignant gliomas in vitro and in vivo.* Hum. Gene Ther. 1998, **9**(11): p. 1599-1608
62. Shinoura N, Koike H, Furitu T, Hashimoto M, Asai A, Kirino T, Hamada H: *Adenovirus-mediated transfer of caspase-*8 *augments cell death in gliomas: implication for gene therapy.* Hum. Gene Ther. 2000, 11(8): p. 1123-1137
63. Shinoura N, Saito K, Yoshida Y, Hashimoto M, Asai A, Kirino T, Hamada H: *Adenovirus-mediated transfer of bax with caspase-8 controlled by myelin basic protein promoter exerts an enhanced cytotoxic effect in gliomas.* Cancer Gene Ther. 2000, 7(5): p. 739-748
64. Jaggi R, Salmons B, Müllener D, Groner B: *The v-mos and H-ras oncogene expression represses glucocorticoid hormone-dependent transcription from the mouse mammary tumor virus LTR.* Embo J. 1986, 5: p. 2609-2616
65. Thackray VG, Lieberman BA, Nordeen SK: *Differential gene induction by glucocorticoid and progesterone receptors.* J. Steroid Biochem. Mol. Biol. 1998, **66**:171-178
66. Behrens C, Hillen W: Gene regulation by tetracyclines: *Constraints of resistance regulation in bacteria shape TetR for application in eukaryotes.* Eur J Biochem 2003, **270:** p. 3109-3121
67. Jiang W, Zhou L, Breyer B, Feng T, Cheng H, Haydon R, Ishikawa A, He TC: *Tetracycline-regulated Gene Expression Mediated by a Novel Chimeric Repressor That Recruits Histone Deacetylases in Mammalian Cells.* J Biol Chem 2001, **276**(48): 45168-45174

**Table 1: Bacterial Strains, Human Cell Lines, Plasmids and Primers**

| **Bacterial Strains** | **Source/Reference** | |
|---|---|---|
| E. *coli* DH10B | Invitrogen | |

| **Human Cell Lines** | **Source/Reference** | |
|---|---|---|
| HeLa | ATCC (No. CCL-2) | |
| 293 | ATCC (No. CRL-1573) | |

| **Plasmids** | **Source/Reference** | |
|---|---|---|
| pLTR-stp | [64] | |
| pVIII-S105 | [53] | |
| pLS105 | [54] | |
| pcDNA3 | Invitrogen | |
| pEGFP-1 | BD Biosciences | |
| pUHD-holin | present invention | |
| pMMTV-holin | present invention | |
| pMMTVb.LTR | present invention | |
| pMMTVGFP | present invention | |

| **Primers** | **Nucleotide Sequence** | **SEQ ID Number** |
|---|---|---|
| V13 | 5' AAAAAACCGCGGATGCCAGAAAAACATGACCTGTTGGCC 3' | (SEQ ID No.1) |
| W13 | 5' AAAAAATCTAGATTATTATTGATTTCTACCATCTTCTACTCC 3' | (SEQ ID No. 2) |
| Holin-F2 | 5' CGCCAGTGTGCTGGAATTCT 3' | (SEQ ID No. 3). |
| Holin-R1 | 5' CTGGCAACTAGAAGGCACAG 3' | (SEQ ID No. 4) |
| Holin-R2 | 5' AAGGCACAGTCGAGGCTGAT 3' | (SEQ ID No. 5) |
| Seq-RC-MMTV-1-R | 5' ACGAGGATGTGAGACAAGTG 3' | (SEQ ID No. 6) |

SEQUENCE LISTING
<110> Fonds zur F^rderung der Forschung auf dem Gebiet der
molekularen Virologie und Gentherapie
<120> Bacteriophage and Prophage Proteins in Cancer Gene
Therapy
<130> P-FON-06-PCT
<160> 9
<170> PatentIn version 3.1

<210> SEQ ID No 1
<211> 39
<212> DNA
<213> Artificial Sequence
<220>
<223> Primer
<400> 1
aaaaaaccgc ggatgccaga aaaacatgac ctgttggcc 39

<210> SEQ ID No 2
<211> 42
<212> DNA
<213> Artificial Sequence
<220>
<223> Primer
<400> 2
aaaaaatcta gattattatt gatttctacc atcttctact cc 42

<210> SEQ ID No 3
<211> 20
<212> DNA
<213> Artificial Sequence
<220>
<223> Primer
<400> 3
cgccagtgtg ctggaattct 20

<210> SEQ ID No 4
<211> 20
<212> DNA
<213> Artificial Sequence
<220>
<223> Primer
<400> 4
ctggcaacta gaaggcacag 20

<210> SEQ ID No 5
<211> 20

<212>  DNA
<213>  Artificial Sequence
<220>
<223>  Primer
<400>  5
aaggcacagt cgaggctgat                    20


<210>  SEQ ID No 6
<211>  20
<212>  DNA
<213>  Artificial Sequence
<220>
<223>  Primer
<400>  6
acgaggatgt gagacaagtg                    20


<170>  PatentIn version 3.1
<210>  SEQ ID No 7
<211>  105
<212>  PRT
<213>  Bacteriophage lambda
<308>  P03705
<313>  (3)..(107)

Met Pro Glu Lys Asn Asp Leu Leu Ala Ala Ile Leu Ala Ala Lys Glu
1               5                   10                  15

Gln Gly Ile Gly Ala Ile Leu Ala Phe Ala Met Ala Tyr Leu Arg Gly
            20                  25                  30

Arg Tyr Asn Gly Gly Ala Phe Thr Lys Thr Val Ile Asp Ala Thr Met
        35                  40                  45

Cys Ala Ile Ile Ala Trp Phe Ile Arg Asp Leu Leu Asp Phe Ala Gly
    50                  55                  60

Leu Ser Ser Asn Leu Ala Tyr Ile Thr Ser Val Phe Ile Gly Tyr Ile
65                  70                  75                  80

Gly Thr Asp Ser Ile Gly Ser Leu Ile Lys Arg Phe Ala Ala Lys Lys
                85                  90                  95

Ala Gly Val Glu Asp Gly Arg Asn Gln
            100                 105


SEQUENCE LISTING
<170>  PatentIn version 3.1
<210>  SEQ ID No 8
<211>  107

<212>  PRT
<213>  Bacteriophage lambda
<308>  P03705
<313>  (1)..(107)

Met Lys Met Pro Glu Lys Asn Asp Leu Leu Ala Ala Ile Leu Ala Ala
1               5                   10                  15

Lys Glu Gln Gly Ile Gly Ala Ile Leu Ala Phe Ala Met Ala Tyr Leu
            20                  25                  30

Arg Gly Arg Tyr Asn Gly Gly Ala Phe Thr Lys Thr Val Ile Asp Ala
        35                  40                  45

Thr Met Cys Ala Ile Ile Ala Trp Phe Ile Arg Asp Leu Leu Asp Phe
    50                  55                  60

Ala Gly Leu Ser Ser Asn Leu Ala Tyr Ile Thr Ser Val Phe Ile Gly
65                  70                  75                  80

Tyr Ile Gly Thr Asp Ser Ile Gly Ser Leu Ile Lys Arg Phe Ala Ala
                85                  90                  95

Lys Lys Ala Gly Val Glu Asp Gly Arg Asn Gln
            100                 105


<170>  PatentIn version 3.1
<210>  SEQ ID No 9
<211>  131
<212>  PRT
<213>  Bacteriophage phi-29
<308>  P11188
<313>  (1)..(131)

Met Lys Met Ile Ala Trp Met Gln His Phe Leu Glu Thr Asp Glu Thr
1               5                   10                  15

Lys Leu Ile Tyr Trp Leu Thr Phe Leu Met Val Cys Met Val Val Asp
            20                  25                  30

Thr Val Leu Gly Val Leu Phe Ala Lys Leu Asn Pro Asn Ile Lys Phe
        35                  40                  45

Ser Ser Phe Lys Ile Lys Thr Gly Val Leu Ile Lys Val Ser Glu Met
    50                  55                  60

Ile Leu Ala Leu Leu Ala Ile Pro Phe Ala Val Pro Phe Pro Ala Gly
65                  70                  75                  80

Leu Pro Leu Leu Tyr Thr Val Tyr Thr Ala Leu Cys Val Ser Glu Ile
                85                  90                  95

Tyr Ser Ile Phe Gly His Leu Arg Leu Val Asp Asp Lys Ser Asp Phe
            100                 105                 110

Leu Glu Ile Leu Glu Asn Phe Phe Lys Arg Thr Ser Gly Lys Asn Lys
        115                 120                 125

Glu Glu Lys
    130

## Claims

1. Use of an isolated bacteriophage-derived protein as medicament for the treatment of a proliferative disease or disorder, wherein the bacteriophage-derived protein induces a cell growth inhibitory activity or cell killing effect in eukaryotic cells.

2. Use according to claim 1, wherein the bacteriophage-derived protein is an alpha-helix-type channel forming protein or at least the functional part or analoga thereof.

3. Use according to claims 1 or 2, wherein the bacteriophage-derived protein is a holin.

4. Use according to claims 1 to 3, wherein the bacteriophage-derived protein is selected from the group consisting of Lambda S105 and Lambda S107 protein (Swiss-Prot Accession Number P03705), the Phi29 GP 14 protein (Swiss-Prot Accession Number P11188), the phage T4 Immunity protein (Swiss-Prot Accession Number P08986), the phage P22 protein 13 (Swiss-Prot Accession Number P09962), the phage P2 TM protein Y (Swiss-Prot Accession Number P51773), the M protein of phage PRD1 (Swiss-Prot Accession Number P27389), the holin protein of phage A118 (Swiss-Prot Accession Number Q37975), the phage A500 holin protein (Swiss-Prot Accession Number Q37977), the DPH holin protein of phage Dp-1 (Swiss-Prot Accession Number 003978), the holin protein of phage HP1 (Swiss-Prot Accession Number P51727), the holin protein of phage rlt (Swiss-Prot Accession Number Q38134), the lysis protein 17.5 of phages T7 (Swiss-Prot Accession Number P03802) and T3 (Swiss-Prot Accession Number P10307), the NucE holin protein of phage P2 Ogr (Swiss-Prot Accession Number Q54418), the holin protein isolated from *Haemophilus somnus* (Swiss-Prot Accession Number Q48281), the P10 protein of phage Phi-6 (Swiss-Prot Accession Number P11127), the protein rV of phage T4 (Swiss-Prot Accession Number P06808), the *Staphylococcus* phage Phi-11 holin (Swiss-Prot Accession Number Q38020), and the *Lactococcus* phage Tuc2009 holin (Swiss-Prot Accession Number Q38613).

5. Use according to claims 1 to 4, wherein the eukaryotic cell is a mammalian and/or human cell.

6. Use according to claims 1 to 5, wherein the bacteriophage-derived protein is transduced into the eukaryotic cell by a eukaryotic expression vector.

7. Use according to claim 6, wherein the eukaryotic expression vector is a viral vector, particularly a retroviral vector, more particularly a replication competent retroviral vector, a ProCon or a ReCon vector.

8. Eukaryotic expression vector useful according to claims 6 or 7 comprising and expressing from an operably linked regulatory element the nucleic acid sequence encoding a bacteriophage-derived protein, which induces a cell growth inhibitory activity or cell killing effect in eukaryotic cells.

9. Eukaryotic expression vector according to claim 8, wherein the vector is a viral vector, particularly a retroviral vector, more particularly a replication competent retroviral vector, a ProCon or a ReCon vector.

10. Nucleic acid sequence comprising the sequence encoding the eukaryotic expression vector according to the claims 8 or 9.

11. A viral particle containing the eukaryotic expression vector according to claims 8 or 9.

12. Eukaryotic cell comprising the nucleic acid sequence according to claim 10.

13. Eukaryotic cell according to claim 12 capable of producing viral particles according to claim 11.

14. A method for introducing the nucleic acid sequence according to claim 10 into an eukaryotic cell, said method comprising the step transducing an eukaryotic cell with the expression vector according to the claims 8 or 9; or infecting an eukaryotic cell with the viral particle according to the claim 11.

15. Use of the expression vector according to the claims 8 or 9, the nucleic acid sequence according to claim 10, the viral particle according to claim 11, the eukaryotic cell according to claim 13 and/or a bacteriophage-derived protein, which induces a cell growth inhibitory activity or cell killing effect as medicament or for the manufacture of a medicament for treatment of cancer diseases.

16. A composition comprising a therapeutically effective amount of the expression vector according to the claims 8 or 9, the nucleic acid sequence according to claim 10, the viral particle according to claim 11, the eukaryotic cell according to claim 13 and/or a bacteriophage-derived protein, which induces a cell growth inhibitory activity or cell killing effect, and a pharmaceutically acceptable carrier.

17. A composition comprising the eukaryotic cell of claim 13 encapsulated in a porous membrane, which allows after administration to a patient a therapeutically effective amount of the viral particles according to claim 11 to be released from the capsules, and a pharmaceutically acceptable carrier.

18. A method for treating cancer cells, comprising administering to a subject the composition according to claim 16 or 17 in a therapeutically effective amount.
